# EUROPEAN PATENT APPLICATION

(11) **EP 4 279 079 A1**
(43) Date of publication of application: **22.11.2023**
(21) Application number: 23174162.0
(22) Date of filing: 18.05.2023
(51) Int. Cl.: A61K 36/9066, A61P 15/08, A61P 15/10, A61K 31/122, A61K 31/385

(54) **NUTRACEUTICAL OR PHARMACEUTICAL COMPOSITION FOR MALE INFERTILITY**

(30) Priority: 18.05.2022 IT 202200010274
(71) Applicant: Neilos S.r.l., 80063 Piano di Sorrento (NA) (IT)
(72) Inventor: DI MAIO, Umberto, 80063 Piano di Sorrento NA (IT)
(74) Representative: Longoni, Alessandra

(57) **Abstract**

The present invention relates to a composition of substances efficient in the prevention and/or treatment of male infertility. The composition of the invention comprises an extract from a plant belonging to the Curcuma genus, alpha lipoic acid and coenzyme Q10. The composition of the invention is prepared in the form of solid, semisolid or liquid pharmaceutical dosage preferably for oral administration.

## Description

The present invention relates to a nutraceutical or pharmaceutical composition efficient in the prevention and/or treatment of male infertility both in humans and animals.

### Background of the invention

Male infertility is a pathology making a man unable to impregnate his partner so without managing to achieve a pregnancy after a period of free unprotected sex longer than a year.

Epidemiological studies carried out on a high number of patients show that about 30% of infertility cases derives from male infertility, 35% of cases is associated with female infertility, 20% of cases is caused by couple infertility while 15% of cases derives from idiopathic infertility due to unknown causes.

The reduced reproductive ability in man derives from anomalies both in the quantity and in the quality of spermatozoa in the ejaculate.

On this basis, several types of male infertility such as azoospermia, oligozoospermia, asthenozoospermia, teratozoospermia, necrospermia, aspermia, hypospermia, leukospermia may be identified.

The term azoospermia means the pathologic condition in which there is total absence of spermatozoa in the ejaculate. Oligozoospermia, asthenozoospermia and teratozoospermia are the more frequent conditions of male infertility and may occur even at the same time in the same patient. Oligozoospermia is the reduced number of spermatozoa present in the sperm, while asthenozoospermia is defined according to the poor motility of spermatozoa and finally teratozoospermia derives from structural alterations of the head and/or tail of male reproductive cells. Instead, necrospermia is the case when spermatozoa are produced but they are not vital and therefore are unable to fertilize the egg cell.

Aspermia is the complete lack of sperm, hypospermia is the case when the ejaculate has a volume lower than 1.5 ml, while leukospermia is the case when white blood cell concentration in the ejaculate is particularly high.

All these types of male infertility derive from different causes which negatively affect sperm production in infertile subjects and may be divided into two main categories such as genetic and acquired ones.

Genetic causes may be congenital in nature when events such as cryptoorchidism with testicle retention, lack of epididymis and anomalies of *vasa deferentia* occur.

Other genetic causes are often associated with mutations such as those characterizing cystic fibrosis transmembrane conductance regulator (CFTR) gene, Klinefelter syndrome, Kallmann syndrome and those resulting in chromosomal anomalies with consequent testicular dysfunction and chromosome Y microdeletions.

In spite of this, the acquired causes are the most representative in male infertility cases and are often associated with varicocele and oxidative stress cases.

Varicocele is due to an anomalous dilation of the veins of the pampiniform plexus of the sperm funnel which blocks the correct flow of spermatozoa in testicles, causing pain and burning both during urination and during the ejaculation process.

In addition, the exposure to toxic chemicals, obesity or an improper life style with drug, smoke and alcohol abuse may result in the production of reactive oxygen (ROS) and nitric oxide (RNS) species causing oxidative stress.

In particular, said pathological event occurs when the main enzymes responsible for the endogenous antioxidant response, such as superoxide dismutase, catalase, glutathione S-transferase and nitric oxide synthase, are unable to control the increase of the ROS and RNS intracellular concentrations. Low concentrations of these molecules are required for the right development of spermatozoa and for their consequent activation during the acrosomal reactions and in the capacitation process near the egg cell.

However, at high concentrations these molecules become unstable and steal electrons from the lipids of the spermatozoa cell membranes inducing lipid peroxidation and impairing their motility and morphology. A prolonged exposure to ROS and RSN may cause severe damages also to the spermatozoa nucleus, resulting in the partial or total fragmentation of their DNA.

The symptoms occurring in infertile patients following the above mentioned different causes relate to swelling and pain sensation at testicles associated with the observed difficulty in urination and ejaculation processes.

In addition, male infertility may also be diagnosed as a function of the causes, thanks to different anatomical and biological analyses. Eventual morphological anomalies of the male urogenital apparatus are identified by the urologist. Thereafter, biological analyses are carried out by sperm withdrawal from infertile patients, to define the sperm parameters by analyzing the spermiogram.

After a well-detailed clinical picture is obtained, the infertile patient can undergo two different treatments, such as ethiological or empirical treatment.

When the cause is known, a targeted treatment is operated to assure the reduction and/or the removal of the problem inducing male infertility.

Examples of ethiological treatments are hormonal therapy with the administration of gonadotropins to promote spermatogenesis or varicocelectomy, a surgery preventing blood reflux into the testicular veins in subjects suffering from varicocele.

When the male infertility cause cannot be diagnosed, an empirical treatment is used trying to improve the lifestyle of the infertile patient through a correct feeding and decreasing the causes coming from environmental and psychological stress.

Also the use of food supplements, which are a very good tool to improve the health conditions of infertile patients, may be associated with all the above. In fact, these latter may restore the normal intracellular levels of ROS and RNS, induce the expression of endogenous antioxidants which are present at low concentrations and so reduce the damages from oxidative stress both at cell membranes and DNA of spermatozoa.

The present invention relates to a nutraceutical or pharmaceutical composition useful for the prevention and/or treatment of male infertility both in humans and animals. The composition object of the present invention comprises an extract from a plant belonging to the Curcuma genus, alpha lipoic acid and coenzyme Q10.

### GLOSSARY

The terms used in the present description are as generally meant by the expert in the field, unless otherwise indicated.

The term "extract", within the present description, means any product connected to a vegetable drug including all the products derived by mechanical treatments (pulverization, grinding, mixing and/or other methods) or by extraction treatments (extraction with solvent, distillation and/or other specific methods) worked on a drug.

### DETAILED DESCRIPTION OF THE INVENTION

Several species belong to the Curcuma genus such as Curcuma longa, Curcuma domestica, Curcuma xanthorrhiza, Curcuma zedoaria.

Among these, the most studied is Curcuma longa.

Curcuma longa plant is commonly known with the name turmeric and belongs to the Zingiberaceae family, native to Southeast Asia and now widely spread in several tropical areas.

C. longa is a perennial, rhizomatous herbaceous plant, preferring sandy or clayish, well-drained soil, growing in areas with temperatures between 25 and 30°C.

The plant may reach up to 1 meter tall, showing oblonged leaves and tubular yellowish flowers. In particular, the pseudo-inflorescences are terminal, spike-shaped with large bracts hiding small-sized flowers.

In traditional Asian cuisine, the fleshy rhizome of C. longa is often used as a spice for the preparation of curry, imparting a typical yellow color to the dish. In addition to this, C. longa is widely used also in the western cuisine to enhance the flavor of dough, meat-based dishes and salades.

C. longa plant is also well known for its several therapeutic effects in treating several pathologies such as cancer, obesity, diabetes mellitus, neurological disorders and cardiovascular diseases.

The beneficial effect resulting from the intake of C. longa mainly depends on the presence of a lipophilic molecule called curcumin in its chemical composition.

Curcumin, which is also named diferuloylmethane, is the main active ingredient which, together with other curcuminoids, constitutes the phytochemical component having pharmacological activity present in the rhizome of C. longa.

Scientific studies carried out on curcumin highlightened in particular its anti-bacterial, anti-cancer, antioxidant and anti-inflammatory properties.

Recently, it was seen that the antioxidant and anti-inflammatory actions of curcumin are very useful for the treatment of idiopathic male infertility.

In a clinical trial carried out on 60 infertile patients to whom curcumin was administered in nano-micelle form, it was reported an improvement of sperm parameters such as sperm count, concentration and motility.

In particular, the antioxidant action of curcumin resulted in a decrease of the excess amount of reactive oxygen species (ROS), restoring their normal cellular levels useful for the spermatogenesis process.

This study allowed to note also the ability of curcumin to induce the expression of endogenous antioxidant molecules such as catalase and superoxide dismutase, useful in preventing the peroxidation of lipids which constitute the cell membranes of spermatozoa.

Furthermore, it was seen that curcumin reduces the damages derived from oxidative stress thanks to its remarkable anti-inflammatory activity. In fact, this lipophilic molecule is able to regulate the inflammatory process due to ROS increase, by decreasing the intracellular concentrations of C-reactive protein and alpha tumor necrosis factor (TNF-α).

Defined as GRAS (Generally Recognized As Safe) by the Food and Drug Administration (FDA), curcumin is, therefore, a natural cure useful for the treatment of idiopathic male infertility thanks to its ability to reduce the damages derived from ROS increase, to induce the expression of endogenous antioxidant molecules and to regulate the expression of mediators of the inflammatory process, maintaining the correct cellular function of spermatozoa.

Alpha lipoic acid is also called thioctic acid and is a saturated fatty acid produced by living organisms. This latter acts as co-factor of the dehydrogenase proteins involved in the production of energy at mitochondrial level.

The peculiar form of this amphipathic molecule derives from octanoic acid, which gives a long carbon chain to which an open ring structure having two thiol groups is added to the ends. This structure is also characterized by the presence of a chiral carbon, which allows to distinguish the molecule into two different optical isomers: R-lipoic acid and S-lipoic acid.

In spite of this, the R isomer is the only naturally occurring form, distributed in food and ex novo synthetized by living beings, while the S isomer is produced only by chemical synthesis.

Usually the amount of alpha lipoic acid produced by the human organism is not enough to meet the energetic cellular demand, therefore food is necessary to provide for this deficiency. In fact, this saturated fatty acid can be absorbed by eating red meat, vegetables and fruits.

The beneficial action derived from the absorption of alpha lipoic acid depends on its remarkable antioxidant activity. In fact, scientific studies showed that this molecule is useful in the treatment of several pathologies such as cardiovascular diseases, neuromuscolar disorders, obesity, cancer and male infertility.

In the specific case of male infertility, it was seen that alpha lipoic acid is able to counteract the damages caused by oxidative stress, due to the increase of intracellular concentrations of reactive oxygen species (ROS).

In particular, these unstable molecules steal electrons from membrane phospholipids making the membrane unstable and causing damages also to the genetic makeup of sperm cells following DNA fragmentation processes.

These negative effects due to oxidative stress result then in a worsening of sperm parameters such as motility, morphology and cell concentration.

In the respect, alpha lipoic acid gives protection to spermatozoa cell membranes through two different mechanisms of action.

The first mechanism is based on the ability of alpha lipoic acid to decrease ROS intracellular levels by acting as antioxidant molecule, maintaining the normal physiological structure of cell membranes and so reducing the damages derived from lipid peroxidation.

In this way, alpha lipoic acid is also able to protect the mitochondrial membranes from ROS attack, assuring the production of energy needed for spermatozoa movement.

In particular, clinical trials carried out on patients suffering from idiopathic infertility showed that the intake of alpha lipoic acid reduces the damaged derived from oxidative stress, resulting in an increase of spermatozoa motility and concentration.

The second mechanism of action depends, instead, on its ability to restore the normal intracellular concentrations of endogenous antioxidant molecules.

In fact, it was seen that alpha lipoic acid is able to increase the expression of glutathione S-transferase (GST), an enzyme widely distributed in spermatozoa and involved in processes of cell detoxification.

In infertile patients, a reduced enzymatic activity of GST results in an increase of ROS concentrations with consequent increase of damages derived from oxidative stress.

It was observed by clinical trials that the intake of alpha lipoic acid assures to restore normal GST levels in sperm cells.

Therefore, the high antioxidant activity showed by alpha lipoic acid combined with its ability to induce the expression of endogenous antioxidant molecules make it a very good candidate for the treatment of several types of male infertility, because it offers a good protection degree to cell membranes of spermatozoa preserving them from damages derived from oxidative stress.

Coenzyme Q10 is an organic molecule consisting of a benzoquinone to which ten isoprenyl units linked each other, forming a very long side chain, are associated. This organic compound is also called vitamin Q since it is very similar to vitamin K structure, even if it is not a real vitamin because it is synthetized by the human organism.

This liposoluble molecule is everywhere in the organism and exists in two different biological forms that is in a reduced form named ubiquinol or in an oxidized form named ubiquinone.

Coenzyme Q10 is mainly located at the mitochondrial cristae, where it plays its main role of electron transporter, required for the production of energy during the oxidative phosphorylation process. In fact, it transfers the electrons derived from complex I and II to complex III making it stable and, at the same time, it also regulates the permeability of the mitochondrial membrane.

Furthermore, coenzyme Q10 induces the expression of several proteins involved in metabolism, cell transport and in the reduction of the oxidative stress, which counteract the damages derived from reactive oxygen species (ROS). Therefore, the maintenance of the endogenous levels of coenzyme Q10 is needed for the production of energy at mitochondrial level and for the correct cell functionality.

However, the concentration of coenzyme Q10 in cells may decrease because of the onset of several pathologies such as diabetes mellitus, hypocholesterolemia, hypertension, cancer, neurodegenerative disorders and male infertility.

In the specific case of male infertility, it was observed that the amount of coenzyme Q10 contained in the head of spermatozoa and in seminal plasma is drastically reduced in patients suffering from idiopathic infertility and asthenozoospermia associated with varicocele.

In particular, coenzyme Q10 deficiency and a consequent decrease of its antioxidant activity results in an increase of ROS intracellular levels, primary responsible of morphological and functional alterations of spermatozoa.

In spite of this, it was observed by clinical trials that the administration of 200 mg coenzyme Q10 in infertile patients results in an increase of the sperm parameters in order to improve sperm morphology, motility and count.

In fact, an increase of coenzyme Q10 intracellular concentration allows this antioxidant molecule to preserve the phospholipid double layer of spermatozoa cell membranes from the event of lipid peroxidation, reducing the excess ROS levels.

Furthermore, coenzyme Q10 is able to induce the expression of two endogenous antioxidant enzymes such as catalase and superoxide dismutase, restoring their correct cell function and so assuring higher protection to spermatozoa towards damages derived from oxidative stress.

The combined action of coenzyme Q10 and the endogenous enzymes allows then to protect spermatozoa from ROS, so decreasing both the morphological damages related to head and tail and the number of damaged spermatozoa.

In addition to the improvement of sperm morphology and count, there is associated also an increase of spermatozoa motility, since the increase of coenzyme Q10 intracellular concentration results in higher production of energy by mitochondria, needed for the movement of spermatozoa toward the egg cell during the fertilization process.

Moreover, an additional clinical study carried out on infertile patients showed that the administration of coenzyme Q10 may also regulate sex hormone levels, so promoting the spermatogenesis process.

In this trial it was observed that infertile patients were characterized by high levels of follicle-stimulating hormone (FSH) and luteinizing hormone (LH) and low levels of inhibin B and testosterone. With the intake of coenzyme Q10, a lowering of FSH and LH concentration and a concomitant increase of inhibin B and testosterone was reported, so promoting the correct production of sperm cells.

All these experimental evidences allow then to consider coenzyme Q10 as a very good ally to counteract oxidative stress, thanks to its antioxidant activity and its ability to both induce the expression of endogenous antioxidant enzymes and regulate the hormonal levels in order to promote the correct spermatogenesis process.

The nutraceutical or pharmaceutical composition according to the invention is as defined in the attached claim 1.

Further features and advantages of the invention are defined in the dependent claims. Claims are integral part of the present description.

A detailed description of some preferred embodiments of the invention is provided hereinafter.

As reported, the nutraceutical or pharmaceutical composition of the present invention comprises an extract from a plant belonging to the Curcuma genus, alpha lipoic acid and coenzyme Q10 useful for the prevention and/or treatment of male infertility.

The term "an extract from a plant belonging to the Curcuma genus" in the context of the present description means that the composition may comprise an extract selected among Curcuma longa, Curcuma domestica, Curcuma xanthorrhiza, Curcuma zedoaria.

The present invention represents a prompt and efficient intervention useful for the prevention and/or treatment of male infertility. Such effect is due to the combined action of the substances composing it. The extract from a plant belonging to the Curcuma genus has marked anti-inflammatory and antioxidant activity; alpha lipoic acid increases the level of endogenous antioxidant such as, in particular, GST; coenzyme Q10 decreases ROS, lipid peroxidation and increases the endogenous antioxidant activity in addition to positively modulate the hormonal profile. All these mechanisms improve the various parameters related to male infertility optimizing spermatogenesis process and maintaining sperm parameters at optimal levels.

The synergetic activity of the above active ingredients can be studied by using in vitro and/or in vivo tests able to evaluate the activity of the compositions according to the present invention.

In order to evaluate the antioxidant action of the single substances and their combinations in decreasing the concentration of reactive oxygen species (ROS) and reactive nitric oxide species (RNS), so preventing lipid peroxidation of cell membranes and consequent fragmentation of cell DNA, several in vitro tests can be carried out.

For example, the first test is the spectrophotometric assay with DPPH (2,2-diphenyl-1-picryl-hydrazyl). This free radical, when dissolved in solution, develops a purple color. The addition of an antioxidant molecule stabilizes DPPH and the solution turns to yellow from purple. This phenomenon is analyzed by spectrometry and the antioxidant activity of a specific substance can be assessed depending from the resultant absorbance value.

The second test is carried out on cells in the presence of dichloro-fluorescein diacetate (DCFH-DA). DCFH-DA is absorbed by cells through passive diffusion and then is de-acylated to prevent its cell leakage. In the presence of free radicals, DCFH produces a fluorescence signal. The addition of an antioxidant molecule inhibits the oxidation of DCFH, decreases the produced fluorescence and assesses its activity, for instance, by spectrofluorimetry.

Furthermore, a decrease in oxidative stress can be observed by analyzing the action of endogenous antioxidant molecules contained into the seminal fluid such as superoxide dismutase (SOD) and catalase (CAT).

SOD activity is evaluated, for example, as a function of pyrogallol oxidation. Pyrogallol is a phenol which oxidizes in the presence of superoxide anion. This latter may be dissociated into molecular oxygen and hydrogen peroxide thanks to the action of SOD at alkaline pH. The inhibition of pyrogallol oxidation results in an increase of absorbance at 420 nm, measured by spectrophotometer and expressed as units per minute per liter of seminal fluid.

CAT antioxidant activity is determined on the basis of the decrease of hydrogen peroxide concentration. The enzyme causes the cleavage of hydrogen peroxide molecules into molecular oxygen and water. This phenomenon is analyzed through spectrophotometric assay by measuring the absorbance value at 240 nm and CAT activity is expressed as specific activity equal to enzymatic activity per minute per milliliter of seminal fluid.

Finally, lipid peroxidation phenomenon can be analyzed through TBARS (thiobarbituric acid reactive species) assay, which measures the formation of malondialdheyde (MDA). In fact, MDA production can be determined as a function of the reaction with thiobarbituric acid, in order to generate a new product which absorbs light at 535 nm and its concentration is expressed as nanomoles per milliliter of seminal fluid.

By using patient's sperm fluid, its quality can be evaluated after the treatment with the composition object of the present invention as: spermatozoa number, motility, morphology, ejaculate volume and its viscosity, presence of white blood cells.

Other methods known to the skilled in the art may be used as well to prove the efficacy of the composition object of the present invention for male infertility.

As previously indicated, the nutraceutical or pharmaceutical composition of the present invention is included into a pharmaceutical product or a food supplement, which is formulated in a suitable dosage form, which composition and preparation falls within the expertise of a skilled in the art.

In a preferred embodiment, the extract from a plant belonging to the Curcuma genus is present in the nutraceutical or pharmaceutical composition of the invention in an amount between 1 mg and 7000 mg, preferably between 5 mg and 5000 mg, still more preferably between 10 mg and 3500 mg per single dosage unit.

Alpha lipoic acid is present in the nutraceutical or pharmaceutical composition of the invention in an amount between 1 mg and 6000 mg, preferably between 5 mg and 4500 mg, still more preferably between 10 mg and 3500 mg per single dosage unit.

Coenzyme Q10 is present in an amount between 0.1 mg and 5000 mg, preferably between 0.5 mg and 2000 mg, still more preferably between 1 mg and 1000 mg per single dosage unit.

All the above mentioned preferred embodiments can be combined each other.

The pharmaceutical product or the food supplement comprising the nutraceutical or pharmaceutical composition of the invention is formulated in a preferably oral pharmaceutical form, which can be solid, semisolid or liquid.

A powder, an orosoluble powder, a granulate, a hard capsule, a soft-gel capsule, a tablet, a sachet, a solution, a syrup, a suspension or an emulsion can be cited as an example.

Some non-limiting examples of nutraceutical or pharmaceutical compositions object of the present invention are provided hereinafter. As above indicated, these nutraceutical or pharmaceutical compositions are formulated as pharmaceutical products or food supplements and administered in a suitable oral dosage form.

The following examples are provided for illustrative purposes only and they are not limiting the scope of the invention as defined by the attached claims.

### EXAMPLES

### EXAMPLE 1

### Granulate

| **Active ingredient** | **Amount per single dosage unit** |
|---|---|
| Curcuma longa extract | 150 mg |
| Alpha lipoic acid | 400 mg |
| Coenzyme Q10 | 50 mg |

### EXAMPLE 2

### Tablet

| **Active ingredient** | **Amount per single dosage unit** |
|---|---|
| Curcuma longa extract | 100 mg |
| Alpha lipoic acid | 300 mg |
| Coenzyme Q10 | 20 mg |

### EXAMPLE 3

### Capsule

| **Active ingredient** | **Amount per single dosage unit** |
|---|---|
| Curcuma longa extract | 50 mg |
| Alpha lipoic acid | 200 mg |
| Coenzyme Q10 | 10 mg |

### EXAMPLE 4

### Granulate

| **Active ingredient** | **Amount per single dosage unit** |
|---|---|
| Curcuma longa extract | 150 mg |
| Alpha lipoic acid | 400 mg |
| Coenzyme Q10 | 10 mg |

## Claims

1. Nutraceutical or pharmaceutical composition comprising the combination of an extract of a plant belonging to the genus *Curcuma,* alpha lipoic acid and Coenzyme Q10.

2. Nutraceutical or pharmaceutical composition according to claim 1, for the use in the prevention and/or the treatment of male infertility in both humans and animals.

3. Nutraceutical or pharmaceutical composition according to claims 1 and 2, in which male infertility is associated with: azoospermia and/or oligozoospermia and/or asthenozoospermia and/or teratozoospermia and or necrospermia and/or aspermia and/or hypospermia and/or leukospermia.

4. Nutraceutical or pharmaceutical composition according to claims 1 to 3, in which the extract of a plant belonging to the genus *Curcuma* is *Curcuma longa* or *Curcuma domestica* or *Curcuma xanthorrhiza* or *Curcuma zedoaria.*

5. Nutraceutical or pharmaceutical composition for the use according to claims 1 to 4, comprising an amount of an extract of a plant belonging to the genus *Curcuma* ranging from 1 mg to 7000 mg, preferably ranging from 5 mg to 5000 mg, still more preferably ranging from 10 mg to 3500 mg for a single dosage unit.

6. Nutraceutical or pharmaceutical composition for the use according to any one of claims 1 to 5, comprising alpha lipoic acid in an amount ranging from 1 mg to 6000 mg, preferably ranging from 5 mg to 4500 mg, still more preferably ranging from 10 mg to 3500 mg for a single dosage unit.

7. Nutraceutical or pharmaceutical composition for the use according to any one of claims 1 to 6, comprising coenzyme Q10 in an amount ranging from 0.1 mg to 5000 mg, preferably ranging from 0.5 mg to 2000 mg, still more preferably ranging from 1 mg to 1000 mg for a single dosage unit.

8. Nutraceutical or pharmaceutical composition for the use according to any one of claims 1 to 7, formulated in a liquid, semi-solid or solid oral dosage form.

9. Nutraceutical or pharmaceutical composition for the use according to claim 8, in which the dosage form is a powder, an orosoluble powder, a granulate, a hard capsule, a soft-gel capsule, a tablet, a sachet, a solution, a syrup a suspension or an emulsion.
